# EUROPEAN PATENT APPLICATION

(11) **EP 4 129 352 A1**
(43) Date of publication of application: **08.02.2023**
(21) Application number: 21779020.3
(22) Date of filing: 04.03.2021
(51) Int. Cl.: A61L 31/04, A61B 17/00, A61B 17/12, A61L 31/02, A61L 31/06, A61L 31/14, A61M 25/00

(54) **EMBOLIZATION AGENT**

(30) Priority: 31.03.2020 JP 2020062454
(71) Applicant: TERUMO Kabushiki Kaisha, Tokyo 151-0072 (JP)
(72) Inventor: ITOH, Yuki, Ashigarakami-gun, Kanagawa 259-0151 (JP); SHIBATA, Hideaki, Ashigarakami-gun, Kanagawa 259-0151 (JP); IKUNO, Eri, Ashigarakami-gun, Kanagawa 259-0151 (JP)
(74) Representative: Casalonga
(86) International application number: PCT/JP2021/008411
(87) International publication number: WO 2021/199883

(57) **Abstract**

The present invention provides technology that can ensure excellent visibility when introducing an embolization agent and that reduces the visibility of said agent after introduction. Provided is an embolization agent having a hydrogel which contains a visualizing agent, and a reaction product of an ethylenically unsaturated monomer, a crosslinking agent, and, as necessary, a bifunctional monomer, wherein the swelling ratio of the embolization agent is 5-300 times, and the post-swelling CT number of the embolization agent is 50-300 HU and falls below the pre-swelling CT number of the embolization agent.

## Description

### Technical Field

The present invention relates to an embolic agent and a treatment system using the embolic agent.

### Background Art

In the related art, for treating an aortic disease (aortic aneurysm, aortic dissociation, or the like), artificial blood vessel replacement surgery for replacing a blood vessel of an affected part with an artificial blood vessel has been performed. However, since this surgical method involves chest opening and abdominal opening, there is a problem that the burden (invasiveness) on a patient is large and hospitalization is prolonged. In consideration of such a problem, in recent years, a treatment using a stent graft (stent graft interpolation) has been widely used instead of the artificial blood vessel replacement surgery. In this surgical method, a stent graft is accommodated in a thin catheter and advanced to a site where there is an aneurysm, and the accommodated stent graft is released and expanded from the catheter and indwelled in a site where there is an aneurysm or dissociation (affected part). This stent graft interpolation is a minimally invasive treatment, and has an advantage that the progress of the patient after surgery is good. On the other hand, in the stent graft interpolation, a blood leakage (endoleak) may occur. There are four types of endoleaks, Type I to Type IV, depending on how the blood leaks into an aneurysm. Among these, the endoleak Type II is a type in which the blood flows into the aneurysm from an inferior mesenteric artery, a lumbar artery (branch blood vessel), or the like due to a decrease in pressure in the aneurysm, and may cause a symptom such as expansion of the aneurysm. For such an endoleak Type II, there is known a method of introducing a biocompatible fluid composition as an embolic substance into a blood vessel causing an endoleak through a catheter and forming an embolus in the causative blood vessel before performing stent graft interpolation (JP-T-2002-539853 (corresponding to US 6,303,100 B1)).

### Summary of Invention

However, there are a plurality of blood vessels that cause an endoleak, and it is difficult to embolize all of the blood vessels. In addition, the biocompatible fluid composition described in JP-T-2002-539853 (corresponding to US 6,303,100 B1) contains a contrast agent (paragraph "0015" (corresponding to Col. 4, Lines 1 to 11 in US 6,303,100 B1)) such that the embolus forming process can be visually recognized. However, there is a problem that, after indwelling, the contrasting property becomes an obstacle to impair visibility by a contrast agent separately introduced for confirming a blood flow, and accurate image diagnosis is difficult or impossible.

Accordingly, the invention has been made in view of the above circumstances, and an object of the invention is to provide a technique capable of ensuring good visibility when introducing an embolic agent and reducing visibility after introduction.

The inventors have conducted intensive studies in order to solve the above problems. As a result, the inventors have found that the above problems can be solved by appropriately controlling a swelling ratio of the embolic agent and the visibility after swelling (CT value after swelling, which is a contrasting property index), and have thus completed the invention.

That is, the above object is achieved by an embolic agent containing a hydrogel containing a visualization agent and a reaction product of an ethylenically unsaturated monomer, a crosslinking agent, and, if necessary, a bifunctional macromer. The embolic agent has a swelling ratio of 5 times to 300 times, and a CT value of the embolic agent after swelling is 50 HU to 300 HU, and is lower than a CT value of the embolic agent before swelling.

### Brief Description of Drawings

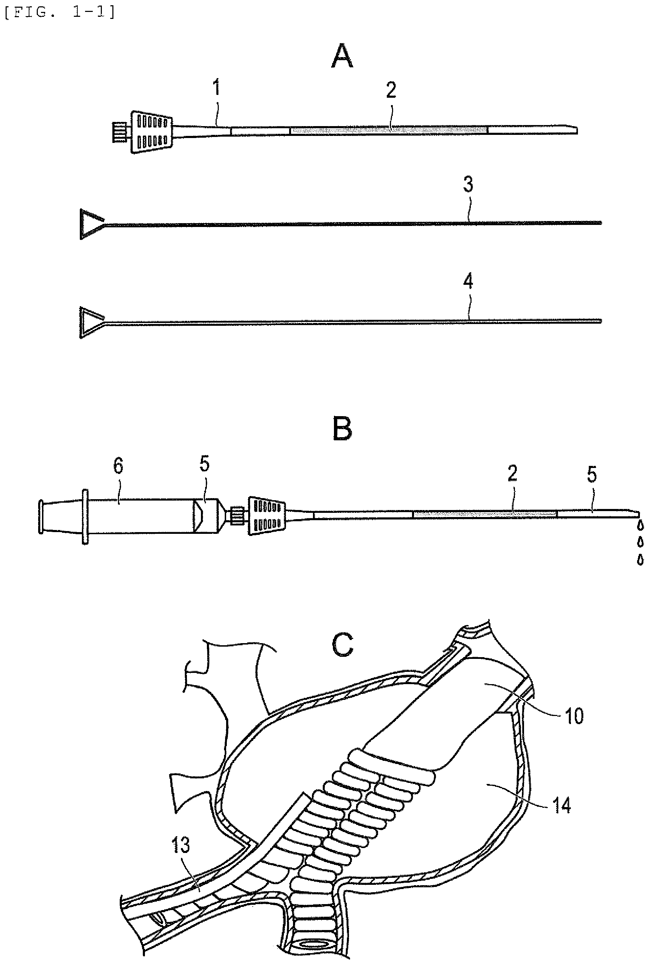

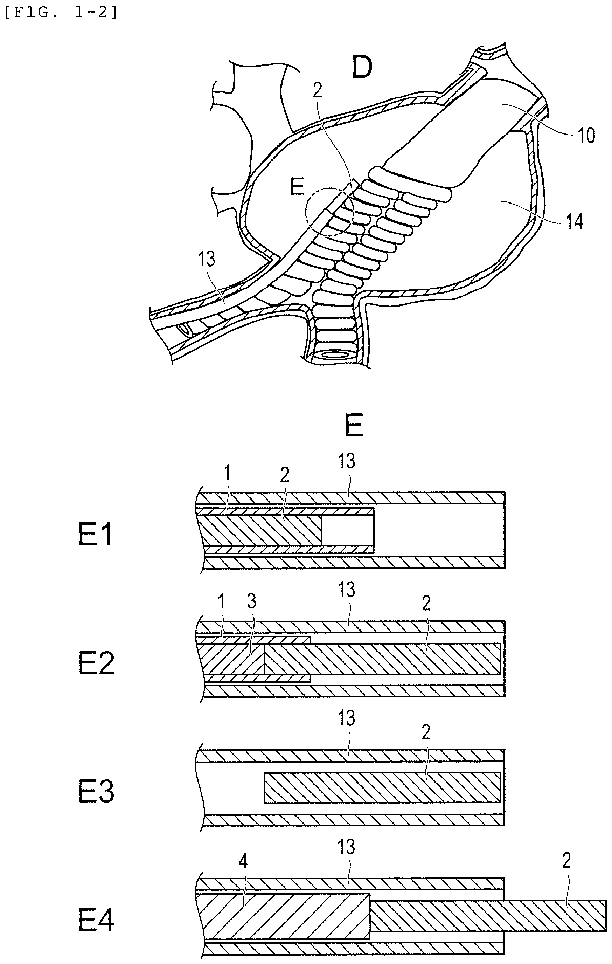

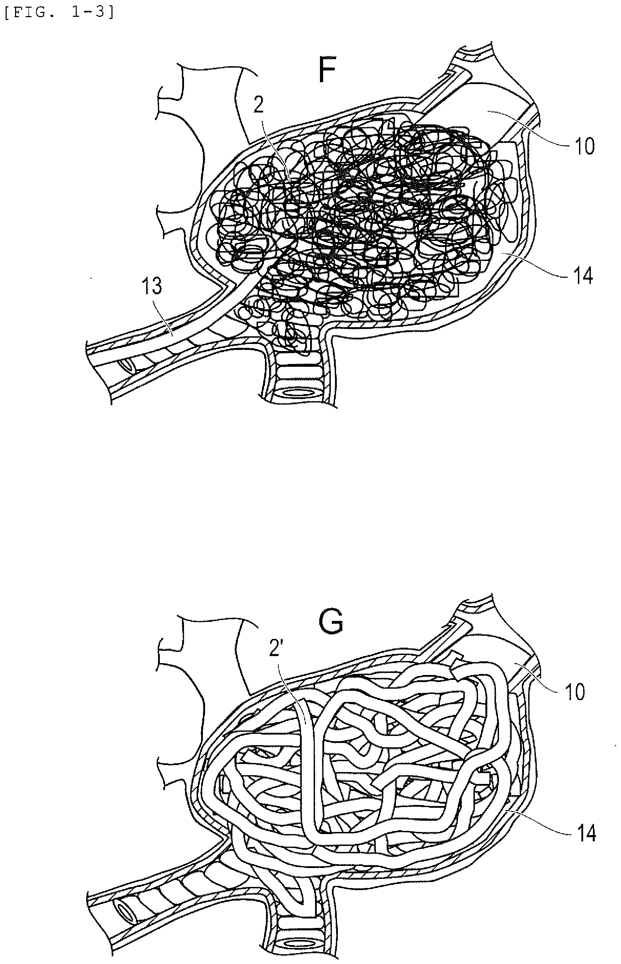

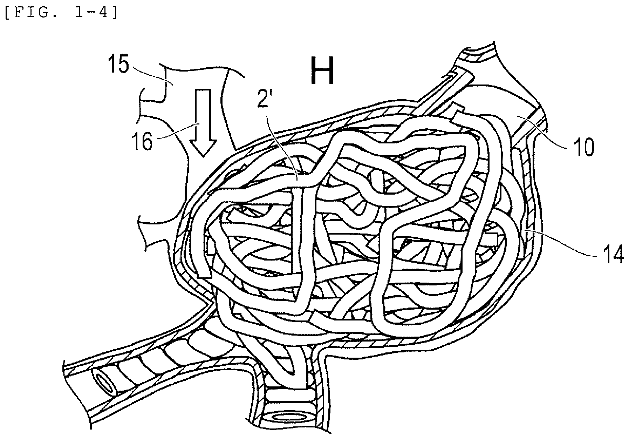

[FIG. 1-1] FIG. 1 is a diagram illustrating a method of using an embolic agent according to an embodiment of the invention.
[FIG. 1-2] FIG. 1 is a diagram illustrating the method of using the embolic agent according to the embodiment of the invention.
[FIG. 1-3] FIG. 1 is a diagram illustrating the method of using the embolic agent according to the embodiment of the invention.
[FIG. 1-4] FIG. 1 is a diagram illustrating the method of using the embolic agent according to the embodiment of the invention.
[FIG. 2-1] FIG. 2 is a diagram illustrating a method of using an embolic agent according to another embodiment of the invention.
[FIG. 2-2] FIG. 2 is a diagram illustrating the method of using the embolic agent according to the another embodiment of the invention.

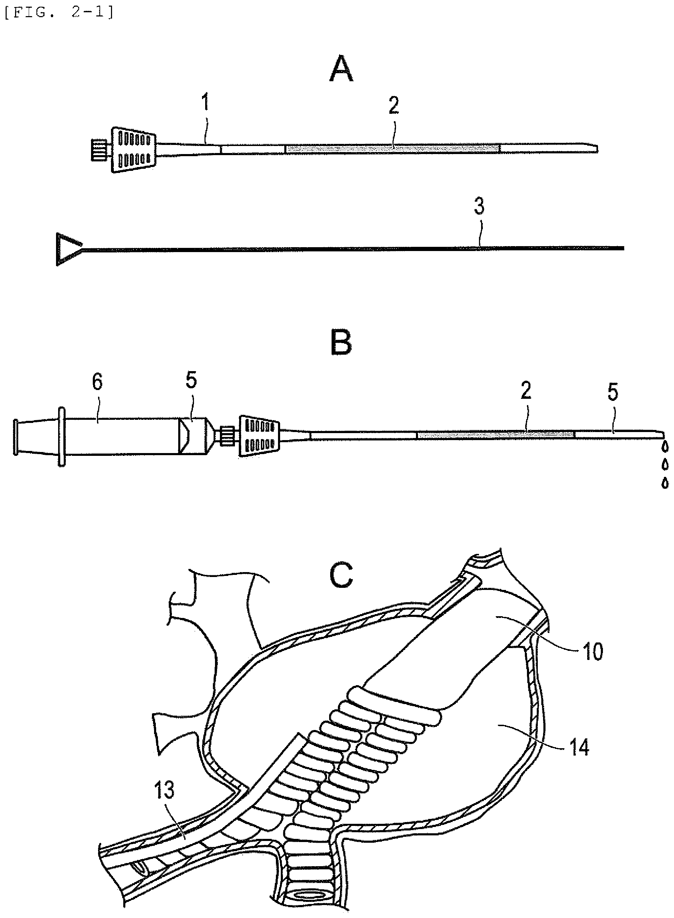

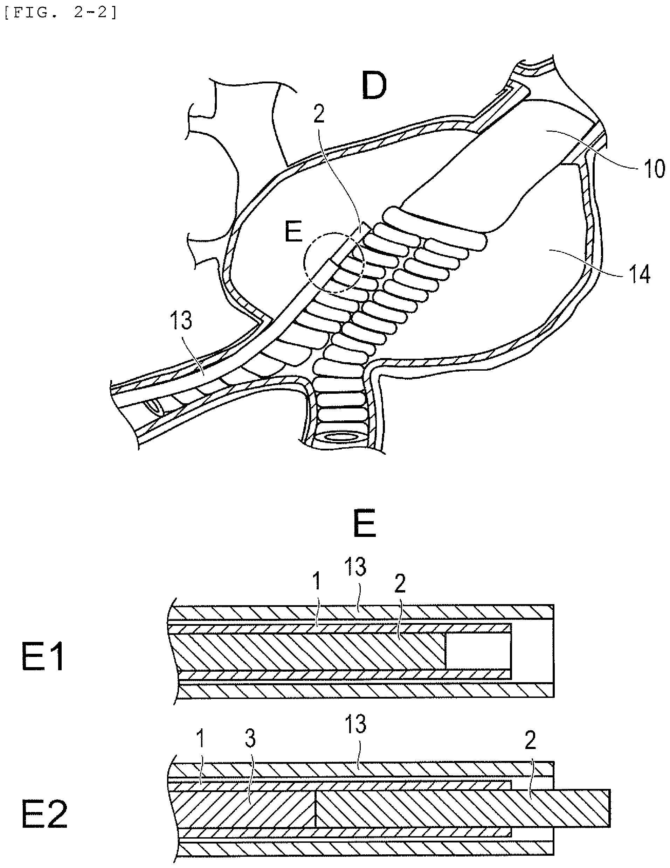

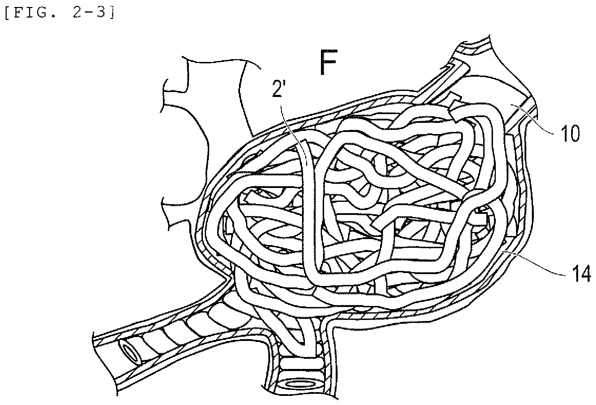

[FIG. 2-3] FIG. 2 is a diagram illustrating the method of using the embolic agent according to the another embodiment of the invention.

### Description of Embodiments

A first aspect of the invention relates to an embolic agent containing a hydrogel containing a visualization agent and a reaction product of an ethylenically unsaturated monomer and a crosslinking agent. The embolic agent has a swelling ratio of 5 times to 300 times, and a CT value of the embolic agent after swelling is 50 HU to 300 HU, and is lower than a CT value of the embolic agent before swelling. According to the embolic agent of the first aspect of the invention, good visibility can be ensured when introducing the embolic agent, and after the introduction, the visibility can be reduced.

A second aspect of the invention relates to an embolic agent containing a hydrogel containing a visualization agent and a reaction product of a bifunctional macromer, an ethylenically unsaturated monomer, and a crosslinking agent. The embolic agent has a swelling ratio of 5 times to 300 times, and a CT value of the embolic agent after swelling is 50 HU to 300 HU, and is lower than a CT value of the embolic agent before swelling. According to the embolic agent of the second aspect of the invention, good visibility can be ensured when introducing the embolic agent, and after the introduction, the visibility can be reduced.

That is, the first and second aspects of the invention relate to an embolic agent containing a hydrogel (preferably composed of a hydrogel filament) containing a visualization agent and a reaction product of an ethylenically unsaturated monomer, a crosslinking agent, and, if necessary, a bifunctional macromer. The embolic agent has a swelling ratio of 5 times to 300 times, and a CT value of the embolic agent after swelling is 50 HU to 300 HU, and is lower than a CT value of the embolic agent before swelling.

According to the embolic agent of the invention, when the embolic agent is introduced, the embolic agent can be satisfactorily visually recognized, and after the embolic agent is introduced into a predetermined site, the visibility of the embolic agent is reduced. Therefore, for example, when the embolic agent is introduced into an aneurysm, a position of the embolic agent can be satisfactorily confirmed until the embolic agent is indwelled in a predetermined site. On the other hand, after the embolic agent is indwelled in the predetermined site, the visibility of the embolic agent is reduced. Therefore, it is possible to clearly confirm the presence or absence of the inflow of a contrast agent into the aneurysm when the contrast agent is introduced through a branch blood vessel (for example, an inferior mesenteric artery, or a lumbar artery) flowing into the aneurysm. Therefore, by using the embolic agent of the invention, it is possible to effectively confirm the presence or absence of an endoleak, particularly an endoleak Type II. Here, a mechanism of exerting the above functions and effects by the configuration of the invention is presumed as follows. The invention is not limited to the following presumption. The embolic agent of the invention has a high swelling ratio of 5 times or more. Therefore, the content (density) of the visualization agent per unit volume after swelling is significantly lower than the content (density) of the visualization agent per unit volume before swelling. Since the embolic agent is not swelled until the embolic agent is indwelled in the predetermined site, the content of the visualization agent per unit volume in the embolic agent is sufficiently high, and thus the position of the embolic agent can be satisfactorily confirmed by appropriate means such as X-rays. On the other hand, after the embolic agent is indwelled in the predetermined site, the embolic agent comes into contact with a body fluid (for example, blood) and swells greatly. Therefore, the content (density) of the visualization agent per unit volume is significantly reduced, and the visibility by appropriate means such as X-rays is significantly reduced. Therefore, the embolic agent hardly or does not interfere with the confirmation of the inflow of the contrast agent into the aneurysm through a blood vessel (for example, an inferior mesenteric artery, or a lumbar artery) that causes an endoleak. Therefore, by using the embolic agent of the invention, it is possible to effectively confirm the presence or absence of an endoleak, particularly an endoleak Type II.

The embolic agent of the invention is disposed in a catheter inner cavity and indwelled in a desired site (for example, an aneurysm) from the catheter inner cavity by a pusher. Therefore, a third aspect of the invention relates to an embolic agent kit. The embolic agent kit includes: the embolic agent according to the first or second aspect; a catheter having an inner cavity, the inner cavity being filled with the embolic agent; and a pusher configured to extrude the embolic agent from the inner cavity of the catheter.

The embolic agent of the invention is particularly effective when combined with stent graft interpolation. Therefore, a fourth aspect of the invention relates to a treatment system including: the embolic agent according to the first or second aspect or the embolic agent kit according to the third aspect; and a graft (particularly, a stent graft).

Hereinafter, embodiments of the invention will be described in detail. The invention is not limited to the following embodiments. In addition, in the present description, "X to Y" indicating a range includes X and Y, and means "X or more and Y or less". In the present description, "X and/or Y" means including at least one of X and Y, and includes "X alone", "Y alone", and "a combination of X and Y". In addition, unless otherwise specified, operations, physical property measurements, and the like are performed under conditions of a room temperature (20°C to 25°C) and a relative humidity of 40% RH to 60% RH.

In the present description, the term "(meth)acrylic" includes both acrylic and methacrylic. Therefore, for example, the term "(meth)acrylic acid" includes both acrylic acid and methacrylic acid. Similarly, the term "(meth)acryloyl" includes both acryloyl and methacryloyl. Therefore, for example, the term "(meth)acryloyl group" includes both an acryloyl group and a methacryloyl group.

### [Embolic Agent]

The embolic agent of the invention contains a hydrogel containing a visualization agent and a reaction product of an ethylenically unsaturated monomer, a crosslinking agent, and, if necessary, a bifunctional macromer, and has a swelling ratio of 5 times to 300 times. The embolic agent has a CT value after swelling of 50 HU to 300 HU, which is lower than a CT value before swelling.

In one embodiment of the invention, the embolic agent is composed of a hydrogel filament containing a visualization agent and a reaction product of an ethylenically unsaturated monomer, a crosslinking agent, and, if necessary, a bifunctional macromer. That is, in one embodiment of the invention, the embolic agent is composed of only the specific hydrogel filament, and does not include other members, for example, a support member (metal support member). In particular, since the embolic agent of the invention does not include the metal support member, halation (artifact, flare, or reflection) derived from a metal does not occur when the embolic agent is observed by appropriate means such as X-rays, and thus the position of the embolic agent can be confirmed more clearly and accurately.

The hydrogel according to the invention may have any form. Examples of the hydrogel include a filament (fiber) shape, a particle shape, a sheet shape, and a strip shape. For example, the embolic agent can be formed into particles by appropriately cutting the hydrogel filament as described above. By filling an elongated catheter with the obtained hydrogel having a particle shape, a filled catheter to be described later can be obtained. In addition, by performing the reaction step in a thin mold rather than in a tube, a hydrogel having a sheet shape or a strip shape can be obtained. The obtained ge1 having a sheet shape or a strip shape is rolled in a short axis direction and inserted into the elongated catheter, whereby the filled catheter to be described later can be obtained. Among these, a filament (fiber) shape is preferred from the viewpoint of easy indwelling in a predetermined site (for example, an aneurysm), the swelling property after indwelling in a predetermined site (for example, an aneurysm), a reduction in a distal embolization risk, or the like. That is, in a preferred embodiment of the invention, the hydrogel is in the form of a filament (fiber).

In addition, the embolic agent according to the invention may contain other components in addition to the hydrogel according to the invention. Here, as the other component, a drug (for example, a blood coagulant) or the like can be used. In addition, when the embolic agent contains other components, the content of the other components is, for example, more than 0 wt% and less than 10 wt% with respect to the total weight of the embolic agent. Preferably, the embolic agent according to the invention is substantially composed of the hydrogel according to the invention. Here, the expression "the embolic agent is substantially composed of the hydrogel" means that the content of the hydrogel in the embolic agent is 95 wt% or more, and is preferably 98 wt% or more (upper limit: 100 wt%). More preferably, the embolic agent according to the invention is composed of the hydrogel according to the invention (the content of the hydrogel in the embolic agent = 100 wt%). Particularly preferably, the embolic agent according to the invention is composed of the hydrogel filament according to the invention (the content of the hydrogel filament in the embolic agent = 100 wt%).

The hydrogel according to the invention swells by contact with an aqueous liquid under a physiological condition. In the present description, the term "physiological condition" means a condition having at least one environmental characteristic in the body or body surface of a mammal (for example, a human). Such characteristics include an isotonic environment, a pH buffering environment, an aqueous environment, a pH near neutral (about 7), a temperature, or a combination thereof. In addition, the term "aqueous liquid" includes, for example, an isotonic solution, water, and a body fluid of a mammal (for example, a human) such as blood, a spinal fluid, plasma, serum, a glass body fluid, and urine.

The embolic agent of the invention has a swelling ratio of 5 times to 300 times. Here, when the swelling ratio is less than 5 times, the density of the visualization agent in the embolic agent after swelling is not sufficiently lowered, and the visibility by appropriate means such as X-rays after the embolic agent is indwelled in the aneurysm cannot be sufficiently reduced. In addition, since the degree of swelling of the embolic agent is low, it is necessary to insert the embolic agent (for example, the hydrogel filament) many times in filling a space between the aneurysm and the stent graft, which takes time and labor. On the other hand, when the swelling ratio exceeds 300 times, the embolic agent is excessively swelled, and it is difficult to determine the introduction amount of the embolic agent (hydrogel) for filling a space between an outer surface of the graft and an inner surface of the aneurysm. From the viewpoint of a further reduction in visibility by appropriate means such as X-rays after the embolic agent is indwelled in the aneurysm, a reduction in strength of the embolic agent, or the like, the swelling ratio of the embolic agent is preferably 50 times to 250 times, more preferably 80 times to 200 times, still more preferably 100 times to 200 times, and particularly preferably 100 times to 180 times. In the present description, the term "swelling ratio" is a value measured by the following method.

### (Measurement of Swelling Ratio)

300 mg of the embolic agent (for example, hydrogel filament) is dried under a reduced pressure for a sufficient time (for example, 24 hours or longer) to obtain a dehydrated sample. The volume (V₀ (mm²)) and the weight (M₀ (g)) of the dehydrated sample are measured. Next, the dehydrated sample is sufficiently (for example, 48 hours or longer) immersed in a sufficient amount (for example, 3000 mL or more) of PBS (pH 7.4) to swell the dehydrated sample, thereby obtaining a swelling sample. The weight (M₁ (g)) of the swelling sample after the immersion is measured. A value ((M₁ - M₀)/V₀) obtained by dividing the amount of change in weight before and after swelling (M₁ (g) - Mo (g)) by the volume (V₀ (mm²)) before swelling is adopted as the swelling ratio (g/mm²).

The embolic agent of the invention shows a CT value after swelling of 50 HU to 300 HU. Here, when the CT value after swelling is less than 50 HU, it is difficult to identify the blood and the blood vessel wall when the embolic agent swells after indwelling. On the other hand, a CT value of the blood vessel drawn with a contrast agent is generally 200 HU to 300 HU. Therefore, when the CT value after swelling exceeds 300 HU, it is difficult to identify the contrast-enhanced blood vessel and the embolic agent from each other, and the inflow of the contrast agent into the aneurysm through the blood vessel (for example, an inferior mesenteric artery, or a lumbar artery) causing an endoleak cannot be satisfactorily confirmed (the presence or absence of the endoleak, particularly, the endoleak Type II cannot be confirmed). From the viewpoint of better confirmation of endoleak, confirmation of the state of the embolic agent (hydrogel) after indwelling, or the like, the CT value of the embolic agent after swelling is more preferably 55 HU to 200 HU, still more preferably 65 HU to 180 HU, and particularly preferably 80 HU or more and less than 135 HU. That is, in a preferred embodiment of the invention, the embolic agent has a swelling ratio of 100 times to 200 times and a CT value after swelling of 65 HU to 180 HU. In the present description, the expression "CT value after swelling" is a visibility (contrasting property) index under X-ray fluoroscopy after swelling, is an image density value (unit: Hounsfield Unit (HU)) measured by the following method, and is represented by a relative value in which the CT value of water and the CT value of air are set to 0 HU and -1000 HU, respectively.

### (Measurement of CT Value after Swelling)

8 cm of the embolic agent (for example, hydrogel filament) is dried under a reduced pressure for a sufficient time (for example, 48 hours) to obtain a dehydrated sample. The dehydrated sample is immersed in a sufficient amount (for example, 1000 mL or more) of PBS (pH 7.4) for 24 hours or longer to swell the dehydrated sample, thereby obtaining a swelling sample.

This swelling sample is placed in a 50 mL centrifuge tube. Next, the 50 mL centrifuge tube containing the embolic agent (hydrogel filament) is laid down on a bed of a medical whole-body X-ray CT diagnostic device (TSX-021B, manufactured by Canon Medical Systems Corporation), and the embolic agent is imaged by the whole-body X-ray CT diagnostic device. CT data is read into analysis software (OsiriX MD, version 6.0, 64 bit, Pixmeo, SARL), and a center slice in a long axis direction of the 50 mL centrifuge tube is displayed. A circular region of interest (ROI) is set in a cross-sectional view of the embolic agent. After the ROI setting is completed, the CT value in the ROI is recorded and used as the CT value after swelling.

In addition, the embolic agent of the invention preferably exhibits a CT value before swelling of 100 HU or more. Accordingly, the embolic agent can be satisfactorily visually recognized during insertion/indwelling, and the embolic agent can be easily identified from blood and a blood vessel wall. From the viewpoint of better visibility of the embolic agent, the CT value before swelling is more preferably 200 HU or more, and particularly preferably more than 300 HU. Accordingly, the visibility of the embolic agent is the same as the visibility of the contrast agent during angiography by X-rays, so that the operator can more easily visually recognize the embolic agent of the invention under X-ray fluoroscopy. An upper limit of the CT value before swelling is preferably about 5000 HU from the viewpoint of easily controlling the CT value after swelling. In the present description, the expression "CT value before swelling" is a visibility (contrasting property) index under X-ray fluoroscopy before swelling, is an image density value (unit: Hounsfield Unit (HU)) measured by the following method, and is represented by a relative value in which the CT value of water and the CT value of air are set to 0 HU and -1000 HU, respectively.

### (Measurement of CT Value before Swelling)

8 cm of the embolic agent (for example, hydrogel filament) is dried under a reduced pressure for a sufficient time (for example, 24 hours) to obtain a dehydrated sample.

This dehydrated sample is placed in a 50 mL centrifuge tube, and the CT value is recorded by the same method as described above (Measurement of CT Value after Swelling), and the CT value is defined as the CT value before swelling.

The size of the hydrogel filament in the case where the embolic agent according to the invention is fibrous (hydrogel filament) is not particularly limited, and can be appropriately selected depending on a size of the aneurysm in which the embolic agent is to be indwelled, the thickness of the branch blood vessel such as an inferior mesenteric artery or a lumbar artery connected to the aneurysm, or the like. For example, a diameter of the hydrogel filament before swelling (in a dry state) is about 0.1 mm to 5 mm, and preferably about 0.2 mm to 3 mm. In addition, a length of the hydrogel filament before swelling (in a dry state) is about 0.5 cm to 200 cm, and preferably about 10 cm to 60 cm. In the present description, the expression "before swelling (in a dry state)" means a state after the embolic agent (for example, the hydrogel filament) is dried under a reduced pressure for a sufficient time (for example, 24 hours).

### (Reaction Product of Ethylenically Unsaturated Monomer and Crosslinking Agent)

### (Reaction Product of Bifunctional Macromer, Ethylenically Unsaturated Monomer, and Crosslinking Agent)

The reaction product constituting the hydrogel filament is a reaction product of an ethylenically unsaturated monomer, a crosslinking agent, and, if necessary, a bifunctional macromer. That is, the reaction product constituting the hydrogel filament is a reaction product of an ethylenically unsaturated monomer and a crosslinking agent (the first aspect) or a reaction product of a bifunctional macromer, an ethylenically unsaturated monomer and a crosslinking agent (the second aspect). Hereinafter, the expression "reaction product of an ethylenically unsaturated monomer and a crosslinking agent" and the expression "reaction product of a bifunctional macromer, an ethylenically unsaturated monomer, and a crosslinking agent" are collectively and simply referred to as a "reaction product".

Here, the ethylenically unsaturated monomer is a monomer having a double bond at a terminal such as an acryloyl group (CH₂=CH-C(=O)-), a methacryloyl group (CH₂=C(CH₃)-C(=O)-), a vinyl group (CH₂=CH-), an acrylamide group (CH₂=CH-C(=O)-NH-), or a methacrylamide group (CH₂=C(CH₃)-C(=O)-NH-). Specific examples of the ethylenically unsaturated monomer include: (meth)acrylic acid, 2-(meth)acryloylethanesulfonic acid, 2-(meth)acryloylpropanesulfonic acid, 2-(meth)acrylamide-2-methylpropanesulfonic acid, vinylsulfonic acid, styrenesulfonic acid, and salts thereof (for example, alkali metal salts, ammonium salts, and amine salts); (meth)acrylamide, N-substituted (meth)acrylamide, 2-hydroxyethyl (meth)acrylate, and 2-hydroxypropyl (meth)acrylate; N,N-dimethylaminoethyl (meth)acrylate, N,N-diethylaminoethyl (meth)acrylate, N,N-diethylaminopropyl (meth)acrylate, and derivatives thereof; N,N-dimethylaminopropyl (meth)acrylamide and quaternized products thereof; and N-vinylpyrrolidinone and derivatives thereof. The ethylenically unsaturated monomers may be used alone or in combination of two or more types thereof. From the viewpoint of higher swelling properties, biocompatibility, non-biodegradability, or the like when in contact with a body fluid, the ethylenically unsaturated monomer is preferably N-vinylpyrrolidinone, 2-hydroxyethyl acrylate, 2-hydroxyethyl methacrylate, and derivatives thereof, and acrylic acid, methacrylic acid, and salts thereof. That is, in a preferred embodiment of the invention, the ethylenically unsaturated monomer is at least one selected from the group consisting of N-vinylpyrrolidinone, 2-hydroxyethyl acrylate, 2-hydroxyethyl methacrylate, derivatives thereof, acrylic acid, methacrylic acid, and salts thereof. In addition, from the viewpoint of further higher swelling properties, biocompatibility, non-biodegradability, or the like when in contact with a body fluid, the ethylenically unsaturated monomer is more preferably (meth)acrylic acid or an alkali metal salt (sodium salt, lithium salt, or potassium salt) thereof, and particularly preferably acrylic acid and/or sodium acrylate.

In addition, the crosslinking agent is not particularly limited as long as the crosslinking agent can crosslink the ethylenically unsaturated monomer or the bifunctional macromer and the ethylenically unsaturated monomer, and a known crosslinking agent can be used. Specific examples of the crosslinking agent include N,N'-methylenebis(meth)acrylamide, (poly)ethylene glycol di(meth)acrylate, 2-hydroxy-3-acryloyloxypropyl (meth)acrylate, 1,10-decanediol di(meth)acrylate, 1,6-hexanediol di(meth)acrylate, 1,9-nonanediol di(meth)acrylate, (poly)propylene glycol di(meth)acrylate, (poly)tetramethylene glycol di(meth)acrylate, and derivatives thereof. These crosslinking agents may be used alone or in combination of two or more types thereof. From the viewpoint of easily controlling the swelling property, biocompatibility, non-biodegradability, or the like when in contact with a body fluid, the crosslinking agent is preferably N,N'-methylenebis(meth)acrylamide, ethylene glycol dimethacrylate, or derivatives thereof. That is, in a preferred embodiment of the invention, the crosslinking agent is at least one selected from the group consisting of N,N'-methylenebisacrylamide, ethylene glycol dimethacrylate, and derivatives thereof. In addition, from the viewpoint of easily controlling the swelling property, biocompatibility, non-biodegradability, or the like when in contact with a body fluid, the crosslinking agent is more preferably N,N'-methylenebis(meth)acrylamide, and particularly preferably N,N'-methylenebisacrylamide.

The bifunctional macromer crosslinks the polymer chain during polymerization to impart softness (flexibility) to the reaction product (therefore, the embolic agent). Therefore, the reaction product (therefore, the embolic agent) containing the bifunctional macromer has excellent followability to a bent portion. Therefore, even when the embolic agent is to be indwelled in the aneurysm via the catheter, the embolic agent can easily pass through the bent portion and be indwelled in the aneurysm. Therefore, the embolic agent of the invention is preferably composed of a hydrogel filament containing a visualization agent and a reaction product of a bifunctional macromer, an ethylenically unsaturated monomer, and a crosslinking agent.

The bifunctional macromer is not particularly limited as long as the bifunctional macromer contains two functional sites, and preferably contains one or more ethylene-based unsaturated groups and two functional sites (a bifunctional ethylene-based unsaturated moldable macromer). Here, the one or more ethylene-based unsaturated groups may form one or both of the functional sites. Examples of the bifunctional macromer include, but are not limited to, polyethylene glycol, polypropylene glycol, poly(tetramethylene oxide), poly(ethylene glycol) diacrylamide, poly(ethylene glycol) dimethacrylamide, poly(ethylene glycol) diacrylate, poly(ethylene glycol) dimethacrylate, poly(propylene glycol) diacrylate, poly(propylene glycol) dimethacrylate, and derivatives thereof. Among these, from the viewpoint of the effect of imparting softness (flexibility) to the embolic agent, the bifunctional macromer is preferably polyethylene glycol, polypropylene glycol, poly(tetramethylene oxide), poly(ethylene glycol) diacrylamide, poly(ethylene glycol) dimethacrylamide, poly(ethylene glycol) diacrylate, poly(ethylene glycol) dimethacrylate, and derivatives thereof. Here, the bifunctional macromers may be used alone or in combination of two or more types thereof. That is, in a preferred embodiment of the invention, the bifunctional macromer is at least one selected from the group consisting of polyethylene glycol, polypropylene glycol, poly(tetramethylene oxide), poly(ethylene glycol) diacrylamide, poly(ethylene glycol) dimethacrylamide, poly(ethylene glycol) diacrylate, poly(ethylene glycol) dimethacrylate, and derivatives thereof. From the viewpoints of biocompatibility and solubility in a solvent, the bifunctional macromer is more preferably poly(ethylene glycol) di(meth)acrylamide. From the viewpoint of decomposability, the bifunctional macromer is more preferably poly(ethylene glycol) di(meth)acrylate.

The molecular weight of the bifunctional macromer is not particularly limited, and is preferably a low molecular weight (bifunctional low molecular weight ethylene-based unsaturated moldable macromer) from the viewpoint of the effect of imparting softness (flexibility) to the embolic agent, improving the swelling ratio, or the like. Specifically, the molecular weight of the bifunctional macromer is preferably about 100 g/mol to about 50,000 g/mol, more preferably about 1,000 g/mol to about 20,000 g/mol, and particularly preferably about 2,000 g/mol to about 15,000 g/mol.

The reaction product according to the invention may contain a structural unit derived from other monomers (other structural units) in addition to the ethylenically unsaturated monomer, the crosslinking agent, and, if necessary, the bifunctional macromer. Here, the other monomers are not particularly limited as long as the other monomers do not inhibit the effects (swelling property, visibility before and after swelling, or the like) of the invention. Specific examples of the monomer include 2,4,6-triiodophenylpenta-4-enoate and 5-(meth)acrylamide-2,4,6-triiodo-n,n'-bis-(2,3-dihydroxypropyl) isophthalamide N-vinylpyrrolidinone. When the reaction product according to the invention has other structural units, the amount (content) of the other structural units is not particularly limited as long as the amount does not inhibit the effects (swelling property, visibility before and after swelling, or the like) of the invention. Specifically, the amount (content) of the other structural units is less than 10 mol%, preferably less than 5 mol%, and more preferably less than 1 mol%, with respect to all structural units constituting the reaction product (lower limit value: more than 0 mol%). When the structural unit derived from other monomers is constituted by two or more types of structural units, a composition of the above structural unit derived from other monomers occupies a proportion (molar ratio (mol%)) of all the structural units derived from other monomers with respect to the total of all the structural units (100 mol%). The mol% is substantially equal to the proportion of the charged amount (mol) of other monomers with respect to the total charged amount (mol) of all the monomers in the production of the reaction product. Particularly preferably, the reaction product does not contain other structural units (the amount (content) of other structural units is 0 mol%) .

The amount (content) of each component in the reaction product is not particularly limited. From the viewpoint of the effect of increasing the swelling property and the mechanical strength, the amount of the ethylenically unsaturated monomer is preferably large. Specifically, for example, when the reaction product is a reaction product of an ethylenically unsaturated monomer and a crosslinking agent, the content of the ethylenically unsaturated monomer is more than 60 wt%, preferably 80 wt% or more and less than 100 wt%, more preferably 90 wt% or more and less than 100 wt%, and particularly preferably 95 wt% or more and less than 100 wt%, with respect to the total weight of the reaction product. In addition, when the reaction product is a reaction product of a bifunctional macromer, an ethylenically unsaturated monomer, and a crosslinking agent, the content of the ethylenically unsaturated monomer is preferably more than 60 wt%, more preferably 63 wt% to 99 wt%, still more preferably 64 wt% or more and less than 98 wt%, and particularly preferably 65 wt% to 97 wt%, with respect to the total weight of the reaction product. Within such a range, the obtained hydrogel filament can exhibit a higher swelling ratio by contact with a body fluid (for example, blood). That is, in a preferred embodiment of the invention, the reaction product of the ethylenically unsaturated monomer and the crosslinking agent contains the ethylenically unsaturated monomer in a proportion of more than 60 wt% with respect to the total weight of the ethylenically unsaturated monomer and the crosslinking agent. In a more preferred embodiment of the invention, the reaction product of the ethylenically unsaturated monomer and the crosslinking agent contains the ethylenically unsaturated monomer in a proportion of 80 wt% or more and less than 100 wt% (more preferably 90 wt% or more and less than 100 wt%, and particularly preferably 95 wt% or more and less than 100 wt%) with respect to the total weight of the ethylenically unsaturated monomer and the crosslinking agent. Alternatively, in a preferred embodiment of the invention, the reaction product of the bifunctional macromer, the ethylenically unsaturated monomer, and the crosslinking agent contains the ethylenically unsaturated monomer in a proportion of more than 60 wt%, with respect to the total weight of the bifunctional macromer, the ethylenically unsaturated monomer, and the crosslinking agent. In a more preferred embodiment of the invention, the reaction product of the bifunctional macromer, the ethylenically unsaturated monomer, and the crosslinking agent contains the ethylenically unsaturated monomer in a proportion of 63 wt% to 99 wt% (more preferably 64 wt% or more and less than 98 wt%, and particularly preferably 65 wt% to 97 wt%) with respect to the total weight of the bifunctional macromer, the ethylenically unsaturated monomer, and the crosslinking agent.

When the reaction product is a reaction product of an ethylenically unsaturated monomer and a crosslinking agent, the content of the crosslinking agent is less than 3 wt%, preferably 0.0001 wt% to 2 wt%, more preferably 0.0005 wt% or more and less than 1 wt%, and particularly preferably 0.001 wt% or more and less than 0.5 wt%, with respect to the total weight of the reaction product. In addition, when the reaction product is a reaction product of a bifunctional macromer, an ethylenically unsaturated monomer, and a crosslinking agent, the content of the crosslinking agent is less than 3 wt%, preferably 0.0001 wt% to 2 wt%, more preferably 0.0005 wt% or more and less than 1 wt%, and particularly preferably 0.001 wt% or more and less than 0.5 wt%, with respect to the total weight of the reaction product. Within such a range, the obtained hydrogel filament can exhibit a higher swelling ratio by contact with a body fluid (for example, blood).

When the reaction product is a reaction product of a bifunctional macromer, an ethylenically unsaturated monomer, and a crosslinking agent, the content of the bifunctional macromer is 40 wt% or less, preferably 1 wt% or more and less than 35 wt%, more preferably more than 2 wt% and less than 20 wt%, and particularly preferably 3 wt% to 15 wt%, with respect to the total weight of the reaction product. Within such a range, the obtained hydrogel filament can exhibit more appropriate softness (flexibility).

In the first aspect, the contents of the ethylenically unsaturated monomer and the crosslinking agent are substantially equal to the proportion of the charged amount (weight) of the corresponding component to the total charged amount (weight) of all components (the ethylenically unsaturated monomer, the crosslinking agent, and other monomers when used) in the production of the reaction product. Similarly, in the second aspect, the contents of the bifunctional macromer, the ethylenically unsaturated monomer, and the crosslinking agent are substantially equal to the proportion of the charged amount (weight) of the corresponding component to the total charged amount (weight) of all components (the bifunctional macromer, the ethylenically unsaturated monomer, the crosslinking agent, and other monomers when used) in the production of the reaction product.

### (Visualization Agent)

The visualization agent constituting the embolic agent (for example, hydrogel filament) is not particularly limited, and may be appropriately selected according to a confirmation method such as X-rays, fluorescent X-rays, ultrasound, a fluorescence method, infrared rays, or ultraviolet rays. Specific examples of the visualization agent include tantalum, barium, and salts thereof (for example, sulfates and carbonates), sodium sulfate, tantalum, tungsten, tungsten carbide, bismuth oxide, platinum or alloys thereof, a cobalt alloy, and gold or an alloy thereof. These visualization agents may be used alone or in combination of two or more types thereof. From the viewpoint of easily controlling the visibility (contrasting property, CT value after swelling) before and after introduction, biocompatibility, or the like, the visualization agent is preferably barium sulfate, platinum, a platinum alloy, gold, or iridium. That is, in a preferred embodiment of the invention, the visualization agent is selected from the group consisting of barium sulfate, platinum, a platinum alloy, gold, and iridium. From the viewpoint of easily controlling the visibility (contrasting property, CT value after swelling) before and after introduction, biocompatibility, or the like, the visualization agent is preferably barium sulfate and platinum, and particularly preferably barium sulfate.

The CT value after swelling can be adjusted by the amount (content; in terms of solid content) of the visualization agent in the embolic agent (for example, hydrogel filament). In addition, from the viewpoint of easily controlling the visibility (contrasting property, CT value after swelling) before and after introduction, a high swelling property, or the like, it is preferable to adjust the amount of the visualization agent in the embolic agent (for example, hydrogel filament). From the above viewpoint, particularly when the visualization agent is barium sulfate, the amount (content; in terms of solid content) of the barium sulfate is preferably more than 6 wt% and less than 50 wt% with respect to the total weight of the reaction product and the barium sulfate. That is, in a preferred embodiment of the invention, the visualization agent is barium sulfate, and is contained in a proportion of more than 6 wt% and less than 50 wt% with respect to the total weight of the reaction product and the visualization agent. From the viewpoint of easily controlling the visibility (contrasting property, CT value after swelling) before and after introduction, a high swelling property, or the like, the amount (content; in terms of solid content) of the barium sulfate is more preferably 9 wt% or more and 47 wt% or less, still more preferably more than 11 wt% and less than 30 wt%, and particularly preferably 17 wt% or more and 19 wt% or less, with respect to the total weight of the reaction product and the visualization agent (barium sulfate). The amount (content) of the visualization agent in the hydrogel filament (embolic agent) is substantially equal to the proportion of the charged amount (wt%) of the visualization agent in the production of the embolic agent.

### (Production of Embolic Agent)

The production of the embolic agent is not particularly limited, and known methods such as those described in JP-T-2011-507637 can be applied in the same manner or appropriately modified. Specifically, when the reaction product is a reaction product of an ethylenically unsaturated monomer and a crosslinking agent (first aspect), preferred is a method (preferred embodiment 1) in which an ethylenically unsaturated monomer, a crosslinking agent, a visualization agent, a solvent, and a reaction initiator and/or a reaction accelerator are mixed (mixing step 1), and the mixture is reacted (polymerization step 1). In addition, when the reaction product is a reaction product of a bifunctional macromer, an ethylenically unsaturated monomer, and a crosslinking agent (second aspect), preferred is a method (preferred embodiment 2) in which a bifunctional macromer, an ethylenically unsaturated monomer, a crosslinking agent, a visualization agent, a solvent, and a reaction initiator and/or a reaction accelerator are mixed (mixing step 2), and the mixture is reacted (polymerization step 2).

Hereinafter, the preferred embodiments will be described. However, the invention is not limited to the following embodiments.

In the mixing step 1 of the preferred embodiment 1, the ethylenically unsaturated monomer, the crosslinking agent, the visualization agent, the solvent, and the reaction initiator and/or the reaction accelerator are mixed. Here, the types and addition amounts of the ethylenically unsaturated monomer, the crosslinking agent, and the visualization agent are not particularly limited, and are the same as those described in the above (Reaction Product of Ethylenically Unsaturated Monomer and Crosslinking Agent) and (Visualization Agent), and thus the description thereof is omitted here.

In addition, in the mixing step 2 of the preferred embodiment 2, the bifunctional macromer, the ethylenically unsaturated monomer, the crosslinking agent, the visualization agent, the solvent, and the reaction initiator and/or the reaction accelerator are mixed. Here, the types and addition amounts of, the bifunctional macromer and the ethylenically unsaturated monomer, the ethylenically unsaturated monomer, the crosslinking agent, and the visualization agent are not particularly limited, and are the same as those described in the above (Reaction Product of Bifunctional Macromer, Ethylenically Unsaturated Monomer, and Crosslinking Agent) and (Visualization Agent), and thus the description thereof is omitted here.

The solvent that can be used in the mixing steps 1 and 2 is not particularly limited as long as the solvent can promote the polymerization in the next step. Specific examples of the solvent include water, methanol, ethanol, isopropyl alcohol (IPA, isopropanol), dichloromethane, and acetone. These solvents may be used alone or in combination of two or more types thereof. The amount of the solvent to be used is not particularly limited, and from the viewpoint of uniformly mixing the components, the total concentration (solid content concentration) of the components is preferably 30 wt% to 70 wt%, and more preferably 35 wt% to 60 wt%.

The reaction initiator that can be used in the mixing steps 1 and 2 is not particularly limited as long as the polymerization reaction can be initiated in the next polymerization steps 1 and 2, and a known reaction initiator can be used. Specific examples of the reaction initiator include N,N,N',N'-tetramethylethylenediamine (TEMED). These reaction initiators may be used alone or in combination of two or more types thereof. The amount of the reaction initiator is not particularly limited as long as the polymerization reaction can be initiated in the next polymerization steps 1 and 2. Specifically, in the mixing step 1, the reaction initiator is preferably about 1.0 × 10⁻³ mol to 3.0 × 10⁻³ mol with respect to 1 mol of the ethylenically unsaturated monomer. In addition, in the mixing step 2, the reaction initiator is preferably about 1.0 × 10⁻³ mol to 3.0 × 10⁻³ mol with respect to 1 mol of the ethylenically unsaturated monomer.

The reaction accelerator that can be used in the mixing steps 1 and 2 is not particularly limited as long as the reaction accelerator can accelerate the polymerization reaction in the next polymerization steps 1 and 2, and a known reaction accelerator can be used. Specific examples of the reaction accelerator include ammonium persulfate (APS), sodium persulfate, benzoyl peroxide, azobisisobutyronitrile (AIBN), and water-soluble AIBN derivatives (for example, 2,2'-azobis(2-methylpropionamidine) dihydrochloride). These reaction accelerators may be used alone or in combination of two or more types thereof. The amount of the reaction accelerator is not particularly limited as long as the polymerization reaction can be promoted in the next polymerization steps 1 and 2. Specifically, in the mixing step 1, the reaction accelerator is preferably about 1.0 × 10⁻⁴ mol to 5.0 × 10⁻⁴ mol with respect to 1 mol of the ethylenically unsaturated monomer. In addition, in the mixing step 2, the reaction accelerator is preferably about 1.0 × 10⁻⁴ mol to 5.0 × 10⁻⁴ mol with respect to 1 mol of the ethylenically unsaturated monomer.

At least one of the reaction initiator and the reaction accelerator may be mixed with the ethylenically unsaturated monomer, the crosslinking agent, or the like, and it is preferable to mix both the reaction initiator and the reaction accelerator from the viewpoint of easily progressing the next polymerization step, a reaction time, or the like.

In the mixing steps 1 and 2, porosigen may be further added. By using the porosigen, pores can be formed in the embolic agent. Therefore, it is possible to enhance the contact with a body fluid and to make the swelling more quickly. Here, the porosigen is not particularly limited, and examples thereof include sodium chloride, ice, sucrose, and sodium bicarbonate. When the porosigen is further used, the addition amount of the porosigen can be appropriately adjusted according to the degree of formation of pores in the embolic agent. For example, the addition amount of the porosigen is about 1 time to 3 times the total charged amount (weight) of the ethylenically unsaturated monomer, the crosslinking agent, and other monomers when used (first aspect), or the total charged amount (weight) of the bifunctional macromer, the ethylenically unsaturated monomer, the crosslinking agent, and other monomers when used (second aspect).

Next, the mixture obtained in the mixing steps 1 and 2 is subjected to a reaction (polymerization or crosslinking reaction is performed) (polymerization steps 1 and 2). Accordingly, the hydrogel filament according to the invention is obtained.

Here, the reaction conditions are not particularly limited as long as the ethylenically unsaturated monomer and the crosslinking agent are sufficiently reacted (polymerized/crosslinked) (polymerization step 1) or the bifunctional macromer, the ethylenically unsaturated monomer, and the crosslinking agent are sufficiently reacted (polymerization step 2), and can be appropriately selected depending on the type and amount of the ethylenically unsaturated monomer, the crosslinking agent, or the like to be used (polymerization step 1) or the bifunctional macromer, the ethylenically unsaturated monomer, the crosslinking agent, or the like to be used (polymerization step 2). Specifically, the reaction temperature is preferably about 10°C to 60°C. In addition, the reaction time is preferably about 1 hour to 6 hours. Under such conditions, the hydrogel filament according to the invention can be produced more efficiently.

In addition, the atmosphere in which the reaction is performed is not particularly limited, and the reaction may be performed in an air atmosphere, an inert gas atmosphere such as nitrogen gas or argon gas, or the like. In addition, the reaction may be performed while stirring the mixture.

In addition, it is preferable to inject the mixture into a tube to perform the reaction. Accordingly, a hydrogel filament having a desired shape and size can be obtained. Alternatively, the reaction may be performed after the tube into which the mixture is injected is wrapped around a mandrel. Accordingly, it is possible to obtain a hydrogel filament having a complicated shape such as a spiral shape or a swirl shape. Here, a material constituting the tube is not particularly limited, and is preferably a material that does not deform at the reaction temperature. Specifically, resins such as polyethylene, polypropylene, and thermoplastic polyether ester elastomer (for example, HYTREL (registered trademark) manufactured by DU PONT-TORAY CO., LTD.) can be used. Since a diameter of the sample after polymerization is reduced by drying the sample in the tube, the sample can be easily taken out by extruding the sample with the mandrel or the like. HYTREL (registered trademark) is excellent in solvent solubility, and thus the reaction product can be easily taken out from the tube after polymerization.

After the reaction, if necessary, the reaction product may be washed to remove the unreacted ethylenically unsaturated monomer, crosslinking agent, and visualization agent (preferred embodiment 1) or the unreacted bifunctional macromer, ethylenically unsaturated monomer, crosslinking agent, and visualization agent (preferred embodiment 2).

Instead of or in addition to the washing step, the reaction product may be incubated in a solution having a low or high pH. In particular, when the ethylenically unsaturated monomer has a carboxyl group (or a group derived from a carboxylate) such as (meth)acrylic acid or a salt thereof, the reaction product is preferably incubated in a solution having a low pH. Accordingly, free protons in the solution protonate the carboxyl group in a hydrogel network. Since the hydrogel filament does not swell until the carboxyl group is deprotonated, the swelling can be controlled. In addition, when the ethylenically unsaturated monomer has an amine group such as N,N-dimethylaminoethyl (meth)acrylate, the reaction product is preferably incubated in a solution having a high pH. Accordingly, the amine group is deprotonated. Since the hydrogel does not swell until the amine group is protonated, the swelling can be controlled. Here, the incubation time, the temperature, and the pH of the solution are not particularly limited, and can be appropriately selected according to a desired degree of swelling (for example, a swelling rate). In general, the incubation time and the temperature are directly proportional to the degree of swelling control, and inversely proportional to the solution pH. In addition, the incubation is preferably performed in a sufficient amount of solution. Accordingly, the hydrogel filament can further swell in the solution. In addition, since a larger number of carboxyl groups can be used for protonation or a larger number of amine groups can be used for deprotonation, the swelling rate can be controlled to a more desired degree. After completion of the incubation, the excess solution is washed and removed, and dehydrated. The hydrogel filament treated with the solution having a low pH can be dehydrated to a smaller size than that of the untreated hydrogel filament. Therefore, in this step, the hydrogel filament can be delivered to a desired site via a catheter having a smaller diameter, so that the invasiveness to the patient can be further prevented, which is preferred.

After the reaction step and/or the washing step and/or the incubation step, the hydrogel filament is dehydrated (dehydrated hydrogel filament). Accordingly, the hydrogel filament (embolic agent) of the invention is obtained. The dehydrated hydrogel filament may be filled in an elongated catheter, packaged, and sterilized into a product form.

### (Use of Embolic Agent)

The embolic agent of the invention has a swelling ratio of 5 times to 300 times. That is, due to a high swelling ratio of 5 times or more, the content (density) of the visualization agent per unit volume after swelling is significantly lower than that before swelling. Therefore, until the embolic agent is indwelled in a predetermined site, the embolic agent can be sufficiently visually recognized by appropriate means such as X-rays, whereas after the embolic agent is indwelled and swells, the visibility of the embolic agent is reduced by appropriate means such as X-rays. Therefore, even after indwelling, the embolic agent hardly or does not interfere with the confirmation of the inflow of the contrast agent into the aneurysm through a blood vessel (for example, an inferior mesenteric artery, or a lumbar artery) causing an endoleak. Therefore, by using the embolic agent of the invention, it is possible to effectively confirm the presence or absence of an endoleak, particularly an endoleak Type II. In addition, after the embolic agent is indwelled in a desired site (for example, an aneurysm), the embolic agent largely swells, so that a void between the desired site and the stent graft can be filled a small number of times. The invention is not limited to the above.

Therefore, it is particularly effective to use the embolic agent of the invention in combination with stent graft interpolation. Therefore, the invention provides the embolic agent according to the first or second aspect to be used in a method for treating an aneurysm. The method includes introducing a graft into an aneurysm of a patient requiring the treatment, and then introducing the embolic agent into a space formed between an inner surface of the aneurysm and an outer surface of the aneurysm. In addition, the invention also provides a treatment system including the embolic agent of the invention and a graft. In the above aspect, the treatment system is used in a method for treating an aneurysm. The method preferably includes introducing a graft into an aneurysm of a patient requiring the treatment, and then introducing the embolic agent of the invention into a space formed between an outer surface of the graft and an inner surface of the aneurysm.

In addition, as will be described in detail below, the embolic agent of the invention is disposed in a catheter inner cavity and indwelled in a desired site (for example, an aneurysm) from the catheter inner cavity by a pusher. Therefore, the invention also provides an embolic agent kit. The embolic agent kit includes: the embolic agent of the invention; a catheter having an inner cavity, the inner cavity being filled with the embolic agent; and an extrusion pusher (elongated extrusion pusher) configured to extrude the embolic agent from the inner cavity of the catheter. Here, the catheter is not particularly limited, and an elongated catheter similar to that generally used for accommodating an embolic agent can be used. Specifically, catheters (elongated bodies) disclosed in JP-A-2015-181673, JP-A-2014-221432, JP-A-2013-198668, or the like can be used. In addition, the invention also provides a treatment system including the embolic agent or the embolic agent kit and a graft.

In addition, as will be described in detail below, a gel-filled catheter in which a catheter inner cavity is filled with the embolic agent of the invention is prepared, and is indwelled in a desired site (for example, an aneurysm) from the catheter inner cavity by a pusher. Therefore, in an embodiment of the invention, there is also provided a treatment system used in a method for treating an aneurysm. The method includes: preparing a gel-filled catheter in which the embolic agent according to the invention is filled in a catheter inner cavity; introducing a ge1 insertion catheter into an aneurysm of a patient requiring the treatment; introducing a graft into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; and inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm.

In an embodiment of the invention, there is also provided a method for treating an aneurysm. The method includes: preparing a gel-filled catheter in which an embolic agent is filled in a catheter inner cavity; introducing a gel insertion catheter into an aneurysm of a patient requiring an aneurysm treatment; introducing a graft into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; and inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm. The embolic agent contains a hydrogel containing a visualization agent and a reaction product of an ethylenically unsaturated monomer and a crosslinking agent, and has a swelling ratio of 5 times to 300 times. The embolic agent has a CT value after swelling of 50 HU to 300 HU, which is lower than a CT value before swelling.

In an embodiment of the invention, there is also provided a method for treating an aneurysm. The method includes: preparing a gel-filled catheter in which an embolic agent is filled in a catheter inner cavity; introducing a gel insertion catheter into an aneurysm of a patient requiring an aneurysm treatment; introducing a graft into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; and inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm. The embolic agent contains a hydrogel containing a visualization agent and a reaction product of a bifunctional macromer, an ethylenically unsaturated monomer, and a crosslinking agent, and has a swelling ratio of 5 times to 300 times. The embolic agent has a CT value after swelling of 50 HU to 300 HU, which is lower than a CT value before swelling.

Hereinafter, the above preferred use will be described with reference to FIG. 1 assuming that a treatment target is an aneurysm. In the invention, the same procedure as the known method disclosed in JP-A-2015-181673, JP-A-2014-221432, JP-A-2013-198668, or the like can be applied except that the embolic agent according to the first or second aspect is used. Therefore, the invention is not limited to the following.

First, a catheter 1 (gel-filled catheter, introducer catheter) in which an inner cavity is filled with an embolic agent (dehydrated state) (for example, a dehydrated hydrogel filament) 2 according to the invention, an extrusion pusher 3, and a delivery pusher 4 are prepared (FIG. 1A). Here, the embolic agent is in a dehydrated state (for example, dehydrated hydrogel filament). A size of the catheter 1 (gel-filled catheter, introducer catheter) is not particularly limited, and is appropriately selected depending on a size of the embolic agent for filling, a size of a site (aneurysm) to be adapted, a size of a gel insertion catheter for indwelling the embolic agent in an aneurysm, or the like. For example, the reaction product (dehydrated hydrogel filament) preferably has a size as described above. Here, the catheter 1 may have a straight shape, or may have a spiral shape in order to compactly accommodate a long gel. A length of the catheter 1 (gel-filled catheter, introducer catheter) is about 3 cm to 500 cm, and preferably 10 cm to 100 cm. With such a length, the gel can be easily extruded from the gel insertion catheter, the number of gels to be inserted into the aneurysm can be reduced, and the efficiency is good. An inner diameter of the catheter 1 (ge1-filled catheter, introducer catheter) is about 0.4 mm to 3.4 mm. An outer diameter of the catheter 1 (gel-filled catheter, introducer catheter) is about 0.5 mm to 3.5 mm. In addition, the extrusion pusher 3 is used to extrude the embolic agent 2 from the catheter 1 into a gel insertion catheter 13 for indwelling the embolic agent 2 in an aneurysm. A size of the extrusion pusher 3 is not particularly limited, and is appropriately selected according to a size of the catheter 1. For example, a length of the extrusion pusher 3 is about 3.5 cm to 501 cm (preferably, 10.5 cm to 101 cm). An outer diameter of the extrusion pusher 3 is preferably about 0.02 mm to 2.0 mm smaller than the inner diameter of the catheter 1 from the viewpoint of easily extruding the embolic agent (dehydrated hydrogel filament) 2. The delivery pusher 4 is used to extrude the embolic agent, which is extruded into the gel insertion catheter 13, from the gel insertion catheter 13 to an aneurysm 14, which will be described in detail below. A size of the delivery pusher 4 is not particularly limited, and is appropriately selected according to a size of the ge1 insertion catheter 13. For example, a length of the delivery pusher 4 is about 20 cm to 1000 cm. An outer diameter of the delivery pusher 4 is preferably about 0.02 mm to 5.0 mm smaller than an inner diameter of the gel insertion catheter 13 from the viewpoint of easily extruding the embolic agent (for example, dehydrated hydrogel filament) 2. A priming solution 5 (for example, physiological saline) is introduced into the inner cavity of the gel-filled catheter 1 through a syringe 6 to remove the air in the catheter inner cavity (priming) (FIG. 1B). When the embolic agent completely fills the inner cavity of the gel-filled catheter 1, priming may be omitted.

Next, the gel insertion catheter 13 is inserted and indwelled into the aneurysm 14 from the femoral artery under X-ray fluoroscopy by a standard intervention procedure. Next, a stent graft 10 is indwelled in the aneurysm 14 of the patient requiring the treatment according to an instruction from a manufacturer (FIG. 1C). In the present embodiment, the gel insertion catheter is introduced before the stent graft is introduced, but the introduction order is not limited to the above embodiment, and the stent graft may be introduced before the ge1 insertion catheter is introduced. That is, in a treatment system (or a method for treating an aneurysm) to be used in a method for treating an aneurysm according to the invention, the method may include: preparing a gel-filled catheter in which the embolic agent is filled in a catheter inner cavity; introducing a graft into an aneurysm of a patient requiring the treatment; introducing the gel insertion catheter into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; and inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm.

The catheter 1 prepared as described above (a gel-filled catheter accommodating the embolic agent (for example, dehydrated hydrogel filament) 2) is inserted into the inner cavity of the gel insertion catheter 13 (FIG. 1E1), and the embolic agent (for example, dehydrated hydrogel filament) 2 is extruded into the inner cavity of the gel insertion catheter 13 using the extrusion pusher 3 (FIG. 1E2). After the catheter 1 is removed (FIG. 1E3), the dehydrated hydrogel filament 2 is extruded, by using the delivery pusher 4, from a front end of the gel insertion catheter 13 into the space formed between the outer surface of the stent graft 10 and the inner surface of the aneurysm 14 (FIGS. 1D and 1E4). Accordingly, the embolic agent (for example, dehydrated hydrogel filament) 2 in a dehydrated state comes into contact with the blood and swells. The above operation is repeated until a swelled embolic agent (for example, swelled hydrogel filament) 2' sufficiently fills the space between the inner surface of the aneurysm 14 and the outer surface of the stent graft 10 (a volume of the completely swelled embolic agent (for example, completely swelled hydrogel filament) 2' ≥ a volume of the space between the inner surface of the aneurysm 14 and the outer surface of the stent graft 10) (FIG. 1F). After confirming that an appropriate amount of the embolic agent 2' in a swelled state is indwelled in the aneurysm 14, the delivery pusher 4 and the gel insertion catheter 13 are removed. Accordingly, the space between the inner surface of the aneurysm 14 and the outer surface of the stent graft 10 is filled with a sufficient amount of the embolic agent 2' in the swelled state (FIG. 1G).

In the above embodiment, the catheter 1 (gel-filled catheter, introducer catheter) may be directly extruded into the aneurysm from the gel insertion catheter 13. That is, according to another embodiment of the invention, there is also provided a treatment system used in a method for treating an aneurysm. The method includes: preparing a gel-filled catheter in which the embolic agent according to the invention is filled in a catheter inner cavity; introducing the gel insertion catheter into an aneurysm of a patient requiring the treatment; introducing a graft into the aneurysm; inserting the gel-filled catheter into an inner cavity of the gel insertion catheter; inserting an extrusion pusher (elongated extrusion pusher) into the inner cavity of the gel-filled catheter; and extruding, by the extrusion pusher, the embolic agent into a space formed between an outer surface of the graft and an inner surface of the aneurysm via the gel insertion catheter.

In another embodiment of the invention, there is also provided a method for treating an aneurysm. The method includes: preparing a gel-filled catheter in which an embolic agent is filled in a catheter inner cavity; introducing a gel insertion catheter into an aneurysm of a patient requiring an aneurysm treatment; introducing a graft into the aneurysm; inserting the gel-filled catheter into an inner cavity of the gel insertion catheter; inserting an extrusion pusher (elongated extrusion pusher) into the inner cavity of the gel-filled catheter; and extruding, by the extrusion pusher, the embolic agent into a space formed between an outer surface of the graft and an inner surface of the aneurysm via the gel insertion catheter. The embolic agent contains a hydrogel containing a visualization agent and a reaction product of an ethylenically unsaturated monomer and a crosslinking agent, and has a swelling ratio of 5 times to 300 times. The embolic agent has a CT value after swelling of 50 HU to 300 HU, which is lower than a CT value before swelling.

In another embodiment of the invention, there is also provided a method for treating an aneurysm. The method includes: preparing a gel-filled catheter in which an embolic agent is filled in a catheter inner cavity; introducing a gel insertion catheter into an aneurysm of a patient requiring an aneurysm treatment; introducing a graft into the aneurysm; inserting the gel-filled catheter into an inner cavity of the gel insertion catheter; inserting an extrusion pusher (elongated extrusion pusher) into the inner cavity of the gel-filled catheter; and extruding, by the extrusion pusher, the embolic agent into a space formed between an outer surface of the graft and an inner surface of the aneurysm via the gel insertion catheter. The embolic agent contains a hydrogel containing a visualization agent and a reaction product of a bifunctional macromer, an ethylenically unsaturated monomer, and a crosslinking agent, and has a swelling ratio of 5 times to 300 times. The embolic agent has a CT value after swelling of 50 HU to 300 HU, which is lower than a CT value before swelling.

Hereinafter, the above embodiment will be described with reference to FIG. 2. In the following description, the description of the same parts as those in FIG. 1 will be omitted.

First, the catheter 1 (gel-filled catheter, introducer catheter) in which the inner cavity is filled with the embolic agent (dehydrated state) (for example, dehydrated hydrogel filament) 2 according to the invention and the extrusion pusher 3 are prepared (FIG. 2A). Next, the priming solution 5 (for example, physiological saline) is introduced into the inner cavity of the gel-filled catheter 1 through the syringe 6 to remove the air in the catheter inner cavity (priming) (FIG. 2B). When the embolic agent completely fills the inner cavity of the gel-filled catheter 1, priming may be omitted. Further, after the gel insertion catheter 13 is inserted and indwelled into the aneurysm 14 from the femoral artery under X-ray fluoroscopy by a standard intervention procedure, the stent graft 10 is indwelled in the aneurysm 14 of the patient requiring the treatment according to an instruction from a manufacturer (FIG. 2C). In the present embodiment, the gel insertion catheter is introduced before the stent graft is introduced, but the introduction order is not limited to the above embodiment, and the stent graft may be introduced before the gel insertion catheter is introduced. Next, the catheter 1 prepared as described above (a gel-filled catheter accomodating embolic agent, (for example, the dehydrated hydrogel filament 2)) is inserted into the vicinity of the front end of the gel insertion catheter 13 (a region extruded by about 1 mm to 2 mm from an end surface of the gel insertion catheter 13 or on a proximal end side) (FIG. 2E1). Further, the embolic agent (for example, dehydrated hydrogel filament) 2 is extruded from the gel insertion catheter 13 using the extrusion pusher 3, and indwelled in the space formed between the outer surface of the stent graft 10 and the inner surface of the aneurysm 14 (FIGS. 2D and 2E2). Accordingly, the embolic agent (for example, dehydrated hydrogel filament) 2 in a dehydrated state comes into contact with the blood and swells. The above operation is repeated until the swelled embolic agent (for example, swelled hydrogel filament) 2' sufficiently fills the space between the inner surface of the aneurysm 14 and the outer surface of the stent graft 10 (a volume of the completely swelled embolic agent (for example, completely swelled hydrogel filament) 2' ≥ a volume of the space between the inner surface of the aneurysm 14 and the outer surface of the stent graft 10). After confirming that an appropriate amount of the embolic agent 2' in a swelled state is indwelled in the aneurysm 14, the extrusion pusher 3 and the gel insertion catheter 13 are removed. Accordingly, the space between the inner surface of the aneurysm 14 and the outer surface of the stent graft 10 is filled with a sufficient amount of the embolic agent 2' in the swelled state (FIG. 2F).

Here, since the embolic agent (for example, dehydrated hydrogel filament) 2 does not come into contact with the blood until indwelled, the visualization agent is present in the embolic agent 2 at a high density. Therefore, until the embolic agent 2 is indwelled in the aneurysm 14, the embolic agent 2 can be satisfactorily visually recognized by appropriate means such as X-ray fluoroscopy. On the other hand, when the embolic agent (for example, dehydrated hydrogel filament) 2 is indwelled in the aneurysm 14, the embolic agent (for example, dehydrated hydrogel filament) 2 comes into contact with the blood in the aneurysm and swells (the embolic agent (for example, swelled hydrogel filament) 2' in a swelled state in FIG. 1G or 2F). In addition, the visualization agent contained in the embolic agent (for example, hydrogel filament) flows out to some extent into the aneurysm due to the blood flow. Therefore, the density of the visualization agent in the embolic agent 2' in the swelled state is reduced. Therefore, the visibility of the embolic agent 2' in the swelled state is reduced. Therefore, when a contrast agent is flowed into the aneurysm 14 via an aorta in order to confirm an endoleak 16 from an inferior mesenteric artery 15 or a lumbar artery, the embolic agent 2' in the swelled state hardly or does not interfere with the confirmation of the inflow and outflow of the contrast agent into and from the aneurysm 14 (FIG. 1H). Therefore, by using the embolic agent of the invention, it is possible to effectively confirm the presence or absence of an endoleak, particularly an endoleak Type II.

That is, according to still another embodiment of the invention, there is also provided a diagnosis system used in a method for diagnosing an endoleak. The method includes: preparing a gel-filled catheter in which the embolic agent according to the invention is filled in a catheter inner cavity; introducing a gel insertion catheter into an aneurysm of a patient requiring a treatment; introducing a graft into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm; and then determining the presence or absence of an endoleak by flowing a contrast agent into the aneurysm.

According to still another embodiment of the invention, there is also provided a diagnosis system used in a method for diagnosing an endoleak. The method includes: preparing a gel-filled catheter in which the embolic agent according to the invention is filled in a catheter inner cavity; introducing the gel insertion catheter into an aneurysm of a patient requiring the treatment; introducing a graft into the aneurysm; inserting the gel-filled catheter into an inner cavity of the gel insertion catheter; inserting an extrusion pusher (elongated extrusion pusher) into the inner cavity of the gel-filled catheter; extruding, by the extrusion pusher, the embolic agent into a space formed between an outer surface of the graft and an inner surface of the aneurysm via the gel insertion catheter; and then determining the presence or absence of an endoleak by flowing a contrast agent into the aneurysm.

In yet still another embodiment of the invention, there is also provided a method for diagnosing an endoleak. The method includes: preparing a gel-filled catheter in which the embolic agent according to the invention is filled in a catheter inner cavity; introducing a gel insertion catheter into an aneurysm of a patient requiring the treatment; introducing a graft into the aneurysm; inserting the gel-filled catheter into an inner cavity of the gel insertion catheter; inserting an extrusion pusher into the inner cavity of the gel-filled catheter, and extruding the embolic agent into the inner cavity of the gel insertion catheter by the extrusion pusher; inserting a delivery pusher into the inner cavity of the gel insertion catheter, and extruding, by the delivery pusher, the embolic agent in the inner cavity of the gel insertion catheter into a space formed between an outer surface of the graft and an inner surface of the aneurysm; and then determining the presence or absence of an endoleak by flowing a contrast agent into the aneurysm.

In yet still another embodiment of the invention, there is also provided a method for diagnosing an endoleak. The method includes: preparing a gel-filled catheter in which the embolic agent according to the invention is filled in a catheter inner cavity; introducing the gel insertion catheter into an aneurysm of a patient requiring the treatment; introducing a graft into the aneurysm; inserting the gel-filled catheter into an inner cavity of the gel insertion catheter; inserting an extrusion pusher (elongated extrusion pusher) into the inner cavity of the gel-filled catheter; extruding, by the extrusion pusher, the embolic agent into a space formed between an outer surface of the graft and an inner surface of the aneurysm via the gel insertion catheter; and then determining the presence or absence of an endoleak by flowing a contrast agent into the aneurysm.

### [Examples]

Effects of the invention will be described with reference to the following Examples and Comparative Examples. However, a technical scope of the invention is not limited to the following Examples. In the following Examples, unless otherwise specified, an operation was performed at room temperature (25°C). Unless otherwise specified, "%" and "parts" mean "wt%" and "parts by weight", respectively.

### Comparative Example 1

3 g of sodium acrylate, 0.375 g of poly(ethylene glycol) dimethacrylamide (molecular weight: 10,000 g/mol), 6 g of barium sulfate, 0.00175 g of N,N'-methylenebisacrylamide, 5 mL of water, 8 g of sodium chloride, 10 µL of tetramethylethylenediamine (TEMED) as a reaction initiator, and 10 µL of an ammonium persulfate (APS) solution (APS concentration: 20 wt%) as a reaction accelerator were mixed to prepare a mixture. The mixture was filled in a polyethylene tube (inner diameter: 3 mm, length: 200 cm), and a polymerization reaction was carried out at room temperature (25°C) for 2 hours. The obtained polymer was taken out from the tube, unreacted substances were removed, and then drying was performed under a reduced pressure to obtain a dehydrated hydrogel filament 1 (an embolic agent 1). The dehydrated hydrogel filament 1 (the embolic agent 1) contains barium sulfate in a proportion of 64 wt%.

The swelling ratio, the CT value before swelling, and the CT value after swelling of the thus obtained embolic agent 1 were measured. The results are shown in Table 1 below.

### Example 1

A dehydrated hydrogel filament 2 (an embolic agent 2) was obtained in the same manner as in Comparative Example 1, except that the amount of barium sulfate was changed to 3 g, unlike in Comparative Example 1. The dehydrated hydrogel filament 2 (the embolic agent 2) contains barium sulfate in a proportion of 47 wt%.

The swelling ratio, the CT value before swelling, and the CT value after swelling of the thus obtained embolic agent 2 were measured. The results are shown in Table 1 below.

### Example 2

A dehydrated hydrogel filament 3 (an embolic agent 3) was obtained in the same manner as in Comparative Example 1, except that the amount of barium sulfate was changed to 1.5 g, unlike in Comparative Example 1. The dehydrated hydrogel filament 3 (the embolic agent 3) contains barium sulfate in a proportion of 31 wt%.

The swelling ratio, the CT value before swelling, and the CT value after swelling of the thus obtained embolic agent 3 were measured. The results are shown in Table 1 below.

### Example 3

A dehydrated hydrogel filament 4 (an embolic agent 4) was obtained in the same manner as in Comparative Example 1, except that the amount of barium sulfate was changed to 0.75 g, unlike in Comparative Example 1. The dehydrated hydrogel filament 4 (the embolic agent 4) contains barium sulfate in a proportion of 18 wt%.

The swelling ratio, the CT value before swelling, and the CT value after swelling of the thus obtained embolic agent 4 were measured. The results are shown in Table 1 below.

### Example 4

A dehydrated hydrogel filament 5 (an embolic agent 5) was obtained in the same manner as in Comparative Example 1, except that the amount of barium sulfate was changed to 0.375 g, unlike in Comparative Example 1. The dehydrated hydrogel filament 5 (the embolic agent 5) contains barium sulfate in a proportion of 10 wt%.

The swelling ratio, the CT value before swelling, and the CT value after swelling of the thus obtained embolic agent 5 were measured. The results are shown in Table 1 below.

### Comparative Example 2

A dehydrated hydrogel filament 6 (an embolic agent 6) was obtained in the same manner as in Comparative Example 1, except that the amount of barium sulfate was changed to 0.1875 g, unlike in Comparative Example 1. The dehydrated hydrogel filament 6 (the embolic agent 6) contains barium sulfate in a proportion of 5 wt%.

The swelling ratio, the CT value before swelling, and the CT value after swelling of the thus obtained embolic agent 6 were measured. The results are shown in Table 1 below.

### Example 5

A dehydrated hydrogel filament 7 (an embolic agent 7) was obtained in the same manner as in Comparative Example 1 except that the amount of poly(ethylene glycol) dimethacrylamide (molecular weight: 10,000 g/mol) was changed to 0.75 g and the amount of barium sulfate was changed to 0.75 g, unlike in Comparative Example 1. The dehydrated hydrogel filament 7 (the embolic agent 7) contains barium sulfate in a proportion of 17 wt%.

The swelling ratio, the CT value before swelling, and the CT value after swelling of the thus obtained embolic agent 7 were measured. The results are shown in Table 1 below.

### Example 6

A dehydrated hydrogel filament 8 (an embolic agent 8) was obtained in the same manner as in Comparative Example 1 except that the amount of poly(ethylene glycol) dimethacrylamide (molecular weight: 10,000 g/mol) was changed to 0.75 g and the amount of barium sulfate was changed to 0.375 g, unlike in Comparative Example 1. The dehydrated hydrogel filament 8 (the embolic agent 8) contains barium sulfate in a proportion of 9 wt%.

The swelling ratio, the CT value before swelling, and the CT value after swelling of the thus obtained embolic agent 8 were measured. The results are shown in Table 1 below.

### Example 7

A dehydrated hydrogel filament 9 (an embolic agent 9) was obtained in the same manner as in Comparative Example 1 except that the amount of poly(ethylene glycol) dimethacrylamide (molecular weight: 10,000 g/mol) was changed to 0.15 g and the amount of barium sulfate was changed to 0.75 g, unlike in Comparative Example 1. The dehydrated hydrogel filament 9 (the embolic agent 9) contains barium sulfate in a proportion of 19 wt%.

The swelling ratio, the CT value before swelling, and the CT value after swelling of the thus obtained embolic agent 9 were measured. The results are shown in Table 1 below.

### Example 8

A dehydrated hydrogel filament 10 (an embolic agent 10) was obtained in the same manner as in Comparative Example 1 except that the amount of poly(ethylene glycol) dimethacrylamide (molecular weight: 10,000 g/mol) was changed to 0.15 g and the amount of barium sulfate was changed to 0.375 g, unlike in Comparative Example 1. The dehydrated hydrogel filament 10 (the embolic agent 10) contains barium sulfate in a proportion of 11 wt%.

The swelling ratio, the CT value before swelling, and the CT value after swelling of the thus obtained embolic agent 10 were measured. The results are shown in Table 1 below.

### Example 9

A dehydrated hydrogel filament 11 (an embolic agent 11) was obtained in the same manner as in Comparative Example 1 except that the amount of barium sulfate was changed to 0.75 g and the amount of sodium chloride was changed to 4 g, unlike in Comparative Example 1. The dehydrated hydrogel filament 11 (the embolic agent 11) contains barium sulfate in a proportion of 18 wt%.

The swelling ratio, the CT value before swelling, and the CT value after swelling of the thus obtained embolic agent 11 were measured. The results are shown in Table 1 below.

### Example 10

A dehydrated hydrogel filament 12 (an embolic agent 12) was obtained in the same manner as in Comparative Example 1 except that the amount of barium sulfate was changed to 0.375 g and the amount of sodium chloride was changed to 4 g, unlike in Comparative Example 1. The dehydrated hydrogel filament 12 (the embolic agent 12) contains barium sulfate in a proportion of 12 wt%.

The swelling ratio, the CT value before swelling, and the CT value after swelling of the thus obtained embolic agent 12 were measured. The results are shown in Table 1 below.

### Example 11

A dehydrated hydrogel filament 13 (an embolic agent 13) was obtained in the same manner as in Comparative Example 1 except that the amount of poly(ethylene glycol) dimethacrylamide (molecular weight: 10,000 g/mol) was changed to 0.15 g, the amount of barium sulfate was changed to 0.75 g, and the amount of sodium chloride was changed to 4 g, unlike in Comparative Example 1. The dehydrated hydrogel filament 13 (the embolic agent 13) contains barium sulfate in a proportion of 19 wt%.

The swelling ratio, the CT value before swelling, and the CT value after swelling of the thus obtained embolic agent 13 were measured. The results are shown in Table 1 below.

### Example 12

A dehydrated hydrogel filament 14 (an embolic agent 14) was obtained in the same manner as in Comparative Example 1 except that the amount of poly(ethylene glycol) dimethacrylamide (molecular weight: 10,000 g/mol) was changed to 0.15 g, the amount of barium sulfate was changed to 0.375 g, and the amount of sodium chloride was changed to 4 g, unlike in Comparative Example 1. The dehydrated hydrogel filament 14 (the embolic agent 14) contains barium sulfate in a proportion of 11 wt%.

The swelling ratio, the CT value before swelling, and the CT value after swelling of the thus obtained embolic agent 14 were measured. The results are shown in Table 1 below.

### Example 13

A dehydrated hydrogel filament 15 (an embolic agent 15) was obtained in the same manner as in Comparative Example 1 except that the amount of barium sulfate was changed to 0.75 g and the amount of sodium chloride was changed to 0 g, unlike in Comparative Example 1. The dehydrated hydrogel filament 15 (the embolic agent 15) contains barium sulfate in a proportion of 18 wt%.

The swelling ratio, the CT value before swelling, and the CT value after swelling of the thus obtained embolic agent 15 were measured. The results are shown in Table 1 below.

### Example 14

A dehydrated hydrogel filament 16 (an embolic agent 16) was obtained in the same manner as in Comparative Example 1 except that the amount of barium sulfate was changed to 0.375 g and the amount of sodium chloride was changed to 0 g, unlike in Comparative Example 1. The dehydrated hydrogel filament 16 (the embolic agent 16) contains barium sulfate in a proportion of 10 wt%.

The swelling ratio, the CT value before swelling, and the CT value after swelling of the thus obtained embolic agent 16 were measured. The results are shown in Table 1 below.

### Example 15

A dehydrated hydrogel filament 17 (an embolic agent 17) was obtained in the same manner as in Comparative Example 1 except that the amount of poly(ethylene glycol) dimethacrylamide (molecular weight: 10,000 g/mol) was changed to 0.15 g, the amount of barium sulfate was changed to 0.75 g, and the amount of sodium chloride was changed to 0 g, unlike in Comparative Example 1. The dehydrated hydrogel filament 17 (the embolic agent 17) contains barium sulfate in a proportion of 19 wt%.

The swelling ratio, the CT value before swelling, and the CT value after swelling of the thus obtained embolic agent 17 were measured. The results are shown in Table 1 below.

### Example 16

A dehydrated hydrogel filament 18 (an embolic agent 18) was obtained in the same manner as in Comparative Example 1 except that the amount of poly(ethylene glycol) dimethacrylamide (molecular weight: 10,000 g/mol) was changed to 0.15 g, the amount of barium sulfate was changed to 0.375 g, and the amount of sodium chloride was changed to 0 g, unlike in Comparative Example 1. The dehydrated hydrogel filament 18 (the embolic agent 18) contains barium sulfate in a proportion of 11 wt%.

The swelling ratio, the CT value before swelling, and the CT value after swelling of the thus obtained embolic agent 18 were measured. The results are shown in Table 1 below.

**[Table 1]**

| | | Amount of barium sulfate (wt%) | Swelling ratio (time) | CT value before swelling (HU) | CT value after swelling (HU) |
|---|---|---|---|---|---|
| Comparative Example 1 | Embolic agent 1 | 64 | 56 | 8561 | 537 |
| Example 1 | Embolic agent 2 | 47 | 104 | 2611 | 252 |
| Example 2 | Embolic agent 3 | 31 | 123 | 2753 | 218 |
| Example 3 | Embolic agent 4 | 18 | 131 | 1466 | 84 |
| Example 4 | Embolic agent 5 | 10 | 148 | 222 | 51 |
| Comparative Example 2 | Embolic agent 6 | 5 | 220 | -351 | 27 |
| Example 5 | Embolic agent 7 | 17 | 118 | 1418 | 91 |
| Example 6 | Embolic agent 8 | 9 | 110 | 315 | 59 |
| Example 7 | Embolic agent 9 | 19 | 65 | 1855 | 123 |
| Example 8 | Embolic agent 10 | 11 | 93 | 892 | 76 |
| Example 9 | Embolic agent 11 | 18 | 131 | 1472 | 132 |
| Example 10 | Embolic agent 12 | 10 | 133 | 603 | 59 |
| Example 11 | Embolic agent 13 | 19 | 89 | 1463 | 112 |
| Example 12 | Embolic agent 14 | 11 | 75 | 590 | 55 |
| Example 13 | Embolic agent 15 | 18 | 119 | 1470 | 131 |
| Example 14 | Embolic agent 16 | 10 | 131 | 595 | 114 |
| Example 15 | Embolic agent 17 | 19 | 86 | 4174 | 144 |
| Example 16 | Embolic agent 18 | 11 | 73 | 1430 | 68 |

### Comparative Example 3

3 g of sodium acrylate, 6 g of barium sulfate, 0.00175 g of N,N'-methylenebisacrylamide, 5 mL of water, 8 g of sodium chloride, 10 µL of tetramethylethylenediamine (TEMED) as a reaction initiator, and 10 µL of an ammonium persulfate (APS) solution (APS concentration: 20 wt%) as a reaction accelerator were mixed to prepare a mixture. The mixture was filled in a polyethylene tube (inner diameter: 3 mm, length: 200 cm), and a polymerization reaction was carried out at room temperature for 2 hours. The obtained polymer was taken out from the tube, unreacted substances were removed, and then drying was performed under a reduced pressure to obtain a dehydrated hydrogel filament 19 (an embolic agent 19). The dehydrated hydrogel filament 19 (the embolic agent 19) contains barium sulfate in a proportion of 67 wt%.

The swelling ratio, the CT value before swelling, and the CT value after swelling of the thus obtained embolic agent 19 were measured. The results are shown in Table 2 below.

### Comparative Example 4

A dehydrated hydrogel filament 20 (an embolic agent 20) was obtained in the same manner as in Comparative Example 3, except that the amount of barium sulfate was changed to 3 g, unlike in Comparative Example 3. The dehydrated hydrogel filament 20 (the embolic agent 20) contains barium sulfate in a proportion of 50 wt%.

The swelling ratio, the CT value before swelling, and the CT value after swelling of the thus obtained embolic agent 20 were measured. The results are shown in Table 2 below.

### Example 17

A dehydrated hydrogel filament 21 (an embolic agent 21) was obtained in the same manner as in Comparative Example 3, except that the amount of barium sulfate was changed to 1.5 g, unlike in Comparative Example 3. The dehydrated hydrogel filament 21 (the embolic agent 21) contains barium sulfate in a proportion of 33 wt%.

The swelling ratio, the CT value before swelling, and the CT value after swelling of the thus obtained embolic agent 21 were measured. The results are shown in Table 2 below.

### Comparative Example 5

A dehydrated hydrogel filament 22 (an embolic agent 22) was obtained in the same manner as in Comparative Example 3, except that the amount of barium sulfate was changed to 0.1875 g, unlike in Comparative Example 3. The dehydrated hydrogel filament 22 (the embolic agent 22) contains barium sulfate in a proportion of 6 wt%.

The swelling ratio, the CT value before swelling, and the CT value after swelling of the thus obtained embolic agent 22 were measured. The results are shown in Table 2 below.

**[Table 2]**

| | | Amount of barium sulfate (wt%) | Swelling ratio (time) | CT value before swelling (HU) | CT value after swelling (HU) |
|---|---|---|---|---|---|
| Comparative Example 3 | Embolic agent 19 | 67 | 35 | 6675 | 1022 |
| Comparative Example 4 | Embolic agent 20 | 50 | 78 | 3552 | 401 |
| Example 17 | Embolic agent 21 | 33 | 107 | 609 | 211 |
| Comparative Example 5 | Embolic agent 22 | 6 | 194 | -508 | 33 |

As can be seen from Tables 1 and 2, in the embolic agents 2 to 5, 7 to 18, and 21 of the invention, the swelling ratio is in the range of 5 times to 300 times, and the CT value after swelling is in the range of 50 HU to 300 HU and is significantly lower than the CT value before swelling. Therefore, it can be seen that when the embolic agent is inserted into the aneurysm, the embolic agent comes into contact with a body fluid such as blood and sufficiently swells, so that the visibility of the embolic agent is reduced, and the embolic agent that has been indwelled and swelled in the aneurysm can be identified from the contrast agent flowing into the aneurysm after the embolic agent has been indwelled and biological tissues such as blood and blood vessel walls. In contrast, in the embolic agents 1, 19, and 20 of Comparative Examples, since the CT value (CT value after swelling) exceeds 300 HU even after swelling, it is presumed that it is difficult to identify the embolic agent that has been indwelled and swelled in the aneurysm from the contrast agent flowing into the aneurysm after the embolic agent has been indwelled. In addition, in the embolic agents 6 and 22 of Comparative Examples, since the CT value after swelling is less than 50 HU, it is presumed that it is difficult to identify the embolic agent that has been indwelled and swelled in the aneurysm from biological tissues such as blood or blood vessel walls. Further, in the embolic agents 6 and 22 of Comparative Examples, since the CT value before swelling is a negative value, the visibility before swelling is remarkably low, and it is presumed that it is difficult to confirm the position of the embolic agent during insertion. In contrast, since the embolic agent of the invention has a sufficiently high CT value before swelling, the embolic agent exhibits sufficient visibility before being inserted into an aneurysm (contact with a body fluid such as blood), and the position of the embolic agent during insertion can be easily confirmed.

From the above results, it can be expected that with the embolic agent of the invention, it is possible to easily confirm the position of the embolic agent during insertion and to clearly confirm the presence of the contrast agent after indwelling.

The present application is based on Japanese Patent Application No. 2020-062454, filed on March 31, 2020, the disclosure of which is referenced and incorporated as a whole.

### Reference Signs List

1: catheter (gel-filled catheter, introducer catheter)
2: embolic agent in dehydrated state (for example, dehydrated hydrogel filament)
3: extrusion pusher
4: delivery pusher
2': embolic agent in swelled state (for example, swelled hydrogel filament)
10: stent graft
13: gel insertion catheter
14: aneurysm
15: inferior mesenteric artery
16: endoleak

## Claims

1. An embolic agent comprising:
a hydrogel containing a visualization agent and a reaction product of an ethylenically unsaturated monomer and a crosslinking agent, wherein
the embolic agent has a swelling ratio of 5 times to 300 times, and
a CT value of the embolic agent after swelling is 50 HU to 300 HU, and is lower than a CT value of the embolic agent before swelling.

2. An embolic agent comprising:
a hydrogel containing a visualization agent and a reaction product of a bifunctional macromer, an ethylenically unsaturated monomer, and a crosslinking agent, wherein
the embolic agent has a swelling ratio of 5 times to 300 times, and
a CT value of the embolic agent after swelling is 50 HU to 300 HU, and is lower than a CT value of the embolic agent before swelling.

3. The embolic agent according to claim 2, wherein the bifunctional macromer is at least one selected from the group consisting of polyethylene glycol, polypropylene glycol, poly(tetramethylene oxide), poly(ethylene glycol) diacrylamide, poly(ethylene glycol) dimethacrylamide, poly(ethylene glycol) diacrylate, poly(ethylene glycol) dimethacrylate, and derivatives thereof.

4. The embolic agent according to any one of claims 1 to 3, wherein the ethylenically unsaturated monomer is at least one selected from the group consisting of N-vinylpyrrolidinone, 2-hydroxyethyl acrylate, 2-hydroxyethyl methacrylate, derivatives thereof, acrylic acid, methacrylic acid, and salts thereof.

5. The embolic agent according to any one of claims 1 to 4, wherein the crosslinking agent is at least one selected from the group consisting of N,N'-methylenebisacrylamide, ethylene glycol dimethacrylate, and derivatives thereof.

6. The embolic agent according to any one of claims 1 to 5, wherein the visualization agent is selected from the group consisting of barium sulfate, platinum, a platinum alloy, gold, and iridium.

7. The embolic agent according to any one of claims 1 to 6, wherein the visualization agent is barium sulfate and is contained in a proportion of more than 6 wt% and less than 50 wt% with respect to a total weight of the reaction product and the visualization agent.

8. The embolic agent according to any one of claims 1 to 7, wherein
the swelling ratio is 100 times to 200 times, and
the CT value after swelling is 65 HU to 180 HU.

9. The embolic agent according to any one of claims 1 to 8, wherein the CT value before swelling is 100 HU or more.

10. The embolic agent according to any one of claims 1 to 9, wherein
the embolic agent is used in a method for treating an aneurysm, and
the method includes introducing a graft into an aneurysm of a patient requiring the treatment, and then introducing the embolic agent into a space formed between an outer surface of the graft and an inner surface of the aneurysm.

11. An embolic agent kit comprising:
the embolic agent according to any one of claims 1 to 9 ;
a catheter having an inner cavity, the inner cavity being filled with the embolic agent; and
an extrusion pusher configured to extrude the embolic agent from the inner cavity of the catheter.

12. A treatment system comprising:
the embolic agent according to any one of claims 1 to 9 or the embolic agent kit according to claim 11; and
a graft.

13. The treatment system according to claim 12, wherein
the treatment system is used in a method for treating an aneurysm, and
the method includes introducing a graft into an aneurysm of a patient requiring the treatment, and then introducing the embolic agent into a space formed between an outer surface of the graft and an inner surface of the aneurysm.

14. The treatment system according to claim 13, wherein the method includes
preparing a gel-filled catheter in which the embolic agent is filled in a catheter inner cavity,
introducing a gel insertion catheter into the aneurysm of the patient requiring the treatment,
introducing the graft into the aneurysm,
inserting the gel-filled catheter into an inner cavity of the gel insertion catheter,
inserting an elongated extrusion pusher into the inner cavity of the gel-filled catheter, and extruding the embolic agent into the inner cavity of the gel insertion catheter by the extrusion pusher, and
inserting an elongated delivery pusher into the inner cavity of the gel insertion catheter, and extruding, by the delivery pusher, the embolic agent in the inner cavity of the gel insertion catheter into the space formed between the outer surface of the graft and the inner surface of the aneurysm.

15. The treatment system according to claim 13, wherein
the method includes
preparing a gel-filled catheter in which the embolic agent is filled in a catheter inner cavity,
introducing a gel insertion catheter into the aneurysm of the patient requiring the treatment,
introducing the graft into the aneurysm,
inserting the gel-filled catheter into an inner cavity of the gel insertion catheter,
inserting an elongated extrusion pusher into the inner cavity of the gel-filled catheter, and
extruding, by the extrusion pusher, the embolic agent into the space formed between the outer surface of the graft and the inner surface of the aneurysm via the gel insertion catheter.
